(19) **Europäisches Patentamt / European Patent Office / Office européen des brevets**

(11) **EP 2 914 777 B1**

(12) **FASCICULE DE BREVET EUROPEEN**

(45) Date de publication et mention
de la délivrance du brevet:
**06.02.2019 Bulletin 2019/06**

(21) Numéro de dépôt: **13795546.4**

(22) Date de dépôt: **28.10.2013**

(51) Int Cl.:
*E01B 35/00* (2006.01)   *E01B 27/12* (2006.01)
*E02D 1/02* (2006.01)   *G01N 3/40* (2006.01)
*G01N 33/42* (2006.01)

(86) Numéro de dépôt international:
**PCT/FR2013/000280**

(87) Numéro de publication internationale:
**WO 2014/068199 (08.05.2014 Gazette 2014/19)**

(54) **PROCÉDÉ D'ÉVALUATION DE LA COMPACITÉ D'UNE COUCHE DE BALLAST DE VOIE FERRÉE ET DISPOSITIFS DE MISE EN OEUVRE DE CE PROCÉDÉ**

VERFAHREN ZUR BEURTEILUNG DER KOMPAKTHEIT EINER SCHOTTERSCHICHT EINER EISENBAHNSTRECKE UND VORRICHTUNGEN ZUR UMSETZUNG DES VERFAHRENS

METHOD FOR EVALUATING THE COMPACTNESS OF A LAYER OF RAILWAY LINE BALLAST AND DEVICES FOR IMPLEMENTING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.10.2012 FR 1202928**

(43) Date de publication de la demande:
**09.09.2015 Bulletin 2015/37**

(73) Titulaires:
• **SNCF Réseau**
**75013 Paris (FR)**
• **Université de Montpellier**
**34090 Montpellier (FR)**
• **Université Blaise Pascal - Clermont-Ferrand II**
**63006 Clermont-Ferrand Cedex (FR)**
• **Centre National de la Recherche Scientifique (C.N.R.S.)**
**75794 Paris Cedex 16 (FR)**

(72) Inventeurs:
• **SAUSSINE, Gilles**
**F-92140 Clamart (FR)**
• **QUEZADA, Juan Carlos**
**F-67000 Strasbourg (FR)**
• **BREUL, Pierre**
**F-63730 Corent (FR)**
• **RADJAI, Franck**
**F-34080 Montpellier (FR)**

(74) Mandataire: **Delaveau, Sophie**
**Lexando & Caracteq**
**14, rue Rodier**
**75009 Paris (FR)**

(56) Documents cités:
**CN-Y- 2 585 882    FR-A1- 2 299 202**
**FR-A5- 2 145 920    US-A- 6 062 090**

EP 2 914 777 B1

**Description**

L'invention concerne principalement un procédé d'évaluation de la compacité d'une couche de ballast de voie ferrée à proximité d'une traverse selon le préambule de la revendication 1.

L'invention porte également sur un dispositif permettant de mettre en oeuvre un tel procédé selon le préambule de la revendication 10 et un dispositif permettant de mettre en oeuvre un tel procédé selon le préambule de la revendication 14.

L'invention porte enfin sur une méthode de prédiction du tassement du ballast d'une voie ferrée incluant une étape d'évaluation de la compacité du ballast.

L'écartement des rails parallèles d'une voie de chemin de fer est maintenu constant par des traverses régulièrement disposées perpendiculairement aux rails. Les traverses permettent en outre de distribuer les efforts transmis par les rails.

Une couche de ballast située sous et entre chaque traverse supporte la voie de chemin de fer. Le ballast est un matériau granulaire provenant du concassage de roches extraites dans des carrières de pierres dures, par exemple de granit, diorite, rhyolite, quartzite ou grès.

Le ballast présente plusieurs fonctions. Notamment, il assure l'ancrage longitudinal et latéral de la voie de chemin de fer. Il amortit par ailleurs les vibrations mécaniques et acoustiques résultant de la circulation des trains. Il transmet en outre les charges appliquées sur les traverses jusqu'à la plate-forme constitué de la couche naturelle du sol, en limitant le tassement de la voie. Enfin, il facilite les opérations de maintenance et de mise en place de la voie et participe au drainage des eaux pluviales.

La circulation des trains sur la voie engendre un tassement progressif du ballast en raison du réarrangement progressif du matériau granulaire constituant le ballast. Ce tassement provoque des défauts sur la voie, se caractérisant notamment par un enfoncement des traverses dans le matériau.

Il est connu de corriger ces défauts par différentes opérations d'entretien et de maintenance. On citera notamment les opérations de bourrage, de calage ou de stabilisation dynamique. Ces opérations permettent de corriger les défauts par vibration et serrage des grains constituant le ballast et situés sous les traverses, ce qui permet de rendre à la voie son profil initial par correction du nivellement longitudinal et transversal.

Des procédés de compactage et de mesure de la densité du ballast sont connus des publications FR2299202 et FR2145920.

Mais ces opérations ne peuvent être réalisées qu'une fois que les défauts deviennent importants. Il en résulte un laps de temps pendant lequel les trains circulent sur des voies déformées.

A ce jour, et à la connaissance des Demanderesses, il n'existe aucune méthode de prédiction du tassement du ballast d'une voie ferrée.

Par ailleurs, l'élaboration d'une méthode de prédiction du tassement du ballast implique nécessairement l'évaluation et la caractérisation du comportement du ballast.

A cet effet, il est connu par la publication FR7602166 de mesurer l'état de densité du ballast d'une voie ferrée. Pour ce faire, on mesure la durée de pénétration dans le ballast d'un outil de pénétration jusqu'à ce qu'il atteigne une profondeur définie. Selon la densité mesurée, on procède ou non aux opérations de bourrage ou de nivellement.

Si cette méthode permet de mesurer à un instant donné l'état du ballast, elle ne permet pas de prédire l'évolution du tassement du ballast dans le temps.

Par ailleurs, le système utilisé pour appliquer cette méthode est nécessairement monté sur une machine mobile de bourrage, nécessite d'être multiplié pour avoir plusieurs points de mesures et doit être alors associé à un système d'acquisition.

Dans ce contexte, la présente invention vise un procédé alternatif d'évaluation de la compacité d'une couche de ballast de voie ferrée à proximité d'une traverse selon la revendication 1, un dispositif de mise en oeuvre de ce procédé selon la revendication 10, et un dispositif de mise en oeuvre de ce procédé selon la revendication 14. permettant d'être monté sur toute machine de maintenance.

L'invention vise également une méthode de prédiction du tassement d'une couche de ballast de voie ferrée.

Le procédé de l'invention peut également comporter les caractéristiques optionnelles suivantes considérées isolément ou selon toutes les combinaisons techniques possibles :

- chaque mesure de résistance de pointe est prise à une distance maximum de cinq centimètres de la traverse et de part et d'autre de la dite traverse.
- les mesures de résistance de pointe sont espacées d'au moins quinze centimètres les unes des autres.
- chaque mesure de résistance de pointe est prise à une distance maximum de cinq centimètres du rail.
- le procédé peut comporter au moins une étape de prise de quatre mesures de la résistance de pointe du ballast à proximité d'une même traverse, chaque mesure étant effectuée du coté extérieur du rail, de part et d'autre de la traverse et à proximité de l'intersection entre la traverse et le rail.
- le procédé peut comporter au moins une étape de prise de huit mesures de la résistance de pointe du ballast à proximité d'une même traverse, quatre première mesures étant effectuées de part et d'autre de la traverse du coté extérieur du rail à proximité de l'intersection entre la traverse et le rail, et quatre secondes mesures étant effectuées du coté intérieur au rail à proximité de l'intersection entre la traverse et le rail.
- chaque mesure de la résistance de pointe du ballast à proximité d'une traverse est réalisée au moyen d'un pénétromètre dynamique comportant une tête de battage sur laquelle est appliquée au moins une chute de masse qui entraîne l'enfoncement dans le ballast d'une pointe conique située à l'extrémité opposée de la tête de battage et reliée à cette dernière par une tige, et un calculateur détermine la résistance de pointe instantanée du ballast par la formule suivante :

$$Qd_i = \frac{E}{A \times e}$$

où $Qd_i$ est la résistance de pointe instantanée pour un battage

$E$ est l'énergie reçue sur la tête de battage exprimée en joules,

$A$ est la section de la pointe exprimée en mètres carrés,

$e$ est la profondeur d'enfoncement de la pointe conique exprimée en mètres,

et le calculateur moyenne toutes les valeurs de résistance de pointe instantanées obtenues pour déterminer la résistance de pointe.

- chaque mesure de la résistance de pointe du ballast à proximité d'une traverse est réalisée au moyen d'un pénétromètre statique pour lequel l'enfoncement du pénétromètre est réalisé au moyen d'un vérin d'enfoncement vertical, et un calculateur détermine la compacité du ballast par la formule suivante :

$$Qd_i = \frac{F}{A}$$

où $Qd_i$ est la résistance de pointe instantanée par enfoncement du pénétromètre,

$A$ est la section de la pointe exprimée en mètres carrés, et

F est la force de poussée exercée sur le pénétromètre exprimée en newton,

et le calculateur moyenne toutes les valeurs de résistance de pointe instantanées obtenues pour déterminer la résistance de pointe.

L'invention porte également sur une méthode de prédiction du tassement d'une couche de ballast de voie ferrée qui est essentiellement caractérisée en ce qu'elle comporte au moins une étape d'évaluation de la compacité d'une couche de ballast de voie ferrée par la détermination de la valeur moyenne de la résistance de pointe du ballast à proximité d'une traverse selon le procédé tel que défini précédemment, et une étape de prédiction du tassement de la dite couche de ballast par application de la formule suivante :

$$\tau_N = \tau_\infty \left( 1 - \frac{1}{1 + B \ln\left(1 + \frac{N}{No}\right)} \right)$$

où $\tau_N$ traduit l'évolution du tassement de la couche de ballast en fonction de $N$ qui est le nombre de passage d'essieux sur la traverse considérée,

où $No$ répond à la formule suivante :

$$No = a \times \ln\left(\frac{Qd_{0\,moy}}{p}\right) + b$$

où $a$ est compris entre 46 et 57, et $b$ est compris entre 40 et 50,

où $Qd_{0\,moy}$ est la résistance de pointe déterminée à l'étape d'évaluation de la compacité de la couche de ballast, et prise à l'issue d'opérations de maintenance ou lors de l'installation de la voie ferrée, et

où $p$ est la pression sous la traverse considérée exprimée en Mégapascal

où $\tau_\infty$ répond à la formule suivante :

$$\tau_\infty = Ho\left(c \times \Gamma + e\right)$$

où $c$ est compris entre 21 et 32, et $e$ est compris entre 0,0040 et 0,006,

où $Ho$ est l'épaisseur de la couche de ballast considérée exprimée en millimètres, et

où $\Gamma$ est l'intensité réduite des vibrations auquel est soumis le ballast à proximité de la traverse considérée et répond à la formule suivante :

$$\Gamma = \frac{A w^2}{\dfrac{p d^2}{m} + g}$$

où $A$ est l'amplitude de l'enfoncement vertical élastique de la voie, calculée à partir d'un modèle mécanique ou mesurée, exprimée en Mètres,

où $w^2 = 2\pi f$, f étant la fréquence de passage des bogies en Hertz

où $p$ est l'effort appliqué par la traverse considérée sur le ballast exprimée en Pascal,

où $d$ est le diamètre moyen d'un grain du ballast exprimée en Mètre, et

où $m$ est la masse d'un grain du ballast exprimée en Kilogramme, et

où $B$ répond à la formule suivante :

$$B = h \times \Gamma^i$$

où $h$ est compris entre 0,0003 et 0,0014, et $i$ est compris entre - 0,3 et - 1,15,
où $\Gamma$ est l'intensité réduite des vibrations auquel est soumis le ballast à proximité de la traverse considérée.

Le dispositif de l'invention peut également comporter les caractéristiques optionnelles suivantes considérées isolément ou selon toutes les combinaisons techniques possibles :

- le dispositif comporte un bâti destiné à être positionné en regard verticalement et à distance de la voie ferrée, et comprenant au moins quatre pénétromètres fixés sur le dit bâti et dont les positions relatives permettent de réaliser quatre mesures de la résistance de pointe du ballast à proximité d'une même traverse.
- la vitesse d'enfoncement de chaque pénétromètre par chaque vérin d'enfoncement vertical est inférieure à 3 centimètres par seconde.
- au moins deux pénétromètres destinés à mesurer la résistance de pointe sur le même coté d'une traverse sont agencés avec un vérin de positionnement latéral qui permet de positionner latéralement les deux pénétromètres considérés.

D'autres caractéristiques et avantages de l'invention ressortiront clairement de la description qui en est donnée ci-dessous, à titre indicatif et nullement limitatif, en référence aux figures annexées parmi lesquelles :

- la figure 1 est une représentation schématique d'un pénétromètre utilisé dans le procédé et le dispositif de l'invention pour déterminer la résistance de pointe du ballast,
- la figure 2 est une représentation schématique de dessus d'une voie ferrée sur laquelle sont symbolisés quatre points de mesure par traverse de la résistance de pointe du ballast selon une première variante du procédé de l'invention,
- la figure 3 une représentation schématique de dessus d'une voie ferrée sur laquelle sont symbolisés huit points de mesure par traverse de la résistance de pointe du ballast selon une seconde variante du procédé de l'invention, et
- la figure 4 est une représentation schématique en perspective du dispositif de l'invention.

La méthode de prédiction du tassement d'une couche de ballast de voie ferrée de l'invention comporte une première étape consistant à évaluer la compacité de la couche de ballast.

Pour ce faire, selon l'invention, au moins deux mesures de la résistance de pointe du ballast à proximité d'une traverse sont réalisées, ces mesures permettant d'évaluer la compacité du ballast sous les traverses.

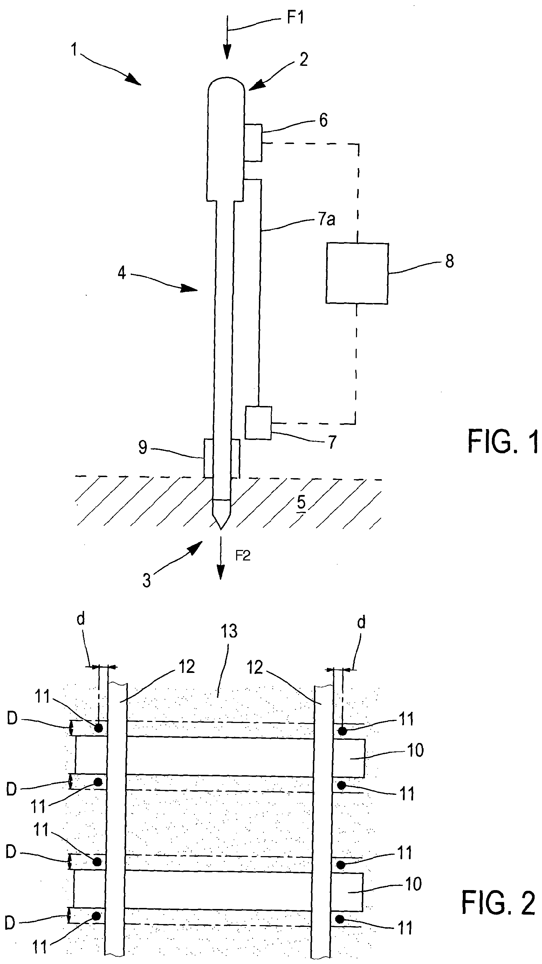

En référence à la figure 1, l'appareil utilisé pour effectuer cette mesure de la résistance de pointe peut être un pénétromètre dynamique du type de celui décrit dans la publication de brevet FR2817344 et commercialisé sous la marque PANDA®

Ce pénétromètre 1 comporte une tête de battage 2 reliée à une pointe conique 3 par un train de tige 4. La pointe conique 3 est destinée à être enfoncée dans le sol 5 par l'action automatique ou d'un opérateur venant frapper sur la tête de battage en direction du sol selon la flèche F1. L'effort transmis à la pointe conique 3 engendre un enfoncement dans le sol selon la flèche F2 plus ou moins important de cette pointe conique 3 selon la compacité du sol.

Le pénétromètre 1 comporte également des jauges de contrainte 6 situées au niveau de la tête de battage 2 qui traduisent l'énergie transmise par l'impact en fonction de la déformation des jauges de contrainte 6.

Le pénétromètre 1 comporte aussi une unité centrale d'acquisition 7 qui est reliée à la tête de battage par une courroie 7a et qui mesure l'enfoncement de la pointe conique 3 dans le sol 5. L'unité centrale d'acquisition 7 et les jauges de contraintes 6 sont reliés à un calculateur 8 qui détermine la résistance de pointe du ballast.

Le fonctionnement du pénétromètre 1 est le suivant. Un opérateur applique un premier coup sur la tête de battage 2 selon la flèche F1. L'énergie transmise par l'impact sur la tête de battage 2 est mesurée à partir de la déformation des jauges de contraintes 6, qui est transmise à l'unité d'acquisition 7.

De façon concomitante, la pointe conique 3 s'enfonce dans le sol 5 selon la flèche F2 d'une profondeur d'enfoncement mesurée par l'unité d'acquisition 7. La profondeur mesurée après l'impact et l'énergie fournie sont transmises au calculateur 8.

Le calculateur détermine alors la résistance de pointe instantanée du ballast par la formule suivante :

$$Qd_i = \frac{E}{A \times e}$$

où $Qd_i$ est la résistance de pointe instantanée pour un battage
$E$ est l'énergie reçue sur la tête de battage, exprimée en joules,
$A$ est la section de la pointe conique exprimée en mètres carrés,
$e$ est la profondeur d'enfoncement de la pointe conique exprimée en mètre.

Puis l'opérateur assène un second coup sur la tête de battage. Le calculateur détermine alors la résistance de pointe instantanée $Qd_i$ associée à ce second coup.

A l'issu des opérations de battage successives, le calculateur 8 moyenne toutes les valeurs de résistance de pointe instantanées $Qd_i$ obtenues pour déterminer la résistance de pointe $Qd$.

La valeur de la résistance de pointe $Qd$ sera d'autant plus précise que le nombre de coups portés sur le pénétromètre est élevé. C'est pourquoi, il est préférable que l'opérateur maîtrise l'enfoncement moyen par coup afin qu'il soit compris entre 5 et 10 millimètres.

Ce pénétromètre dynamique 1 est particulièrement adapté à la mesure de la compacité du ballast de voie ferrée en raison de son encombrement réduit provenant du fait que son fonctionnement repose sur l'application d'une énergie variable, et qu'il est donc possible d'adapter l'énergie fournie sur la tête de battage aux conditions du site de mesures.

En variante, on peut également utiliser un pénétromètre de type statique pour lequel l'enfoncement est réalisé par l'actionnement d'un vérin d'enfoncement vertical. Dans ce cas, la résistance de pointe instantanée $Qd_i$ est déterminée par la formule suivante :

$$Qd_i = \frac{F}{A}$$

où $Qd_i$ est la résistance de pointe instantanée par enfoncement du pénétromètre,

$A$ est la section de la sonde exprimée en Mètres carrés, et

$F$ est la force de poussée exercée sur le pénétromètre exprimée en Newtons.

Comme pour le pénétromètre dynamique, la valeur de la résistance de pointe $Qd$ du ballast est déterminée en moyennant les valeurs des résistances de pointe instantanées $Qd_i$ obtenues lors des enfoncements successifs du pénétromètre.

Il est à noter qu'au regard de la nature du ballast, la vitesse d'enfoncement du pénétromètre statique doit être inférieure à 3 centimètres par seconde. En effet, au-delà d'une vitesse de 3 centimètres par seconde, la surestimation de la résistance de pointe est trop élevée.

Selon le procédé de l'invention, cette mesure de la résistance de pointe $Qd$ du ballast, qu'elle soit réalisée par battage avec un pénétromètre de type dynamique, ou par une force de poussée d'enfoncement avec un pénétromètre de type statique, doit être effectuée en au moins deux points à proximité d'une traverse. Les valeurs de résistance de pointe $Qd$ associées à ces deux points sont ensuite moyennées pour évaluer la résistance de pointe moyenne $Qd_{moy}$ sous une traverse.

Plus précisément et en référence à la figure 2, selon une première variante du procédé de l'invention, quatre mesures de la résistance de pointe $Qd$ sont prises à proximité d'une traverse 10. Sont représentés sur la figure 2 les points d'impact 11 du pénétromètre 1 avec le sol reflétant les mesures de résistance de pointe $Qd$.

Chaque mesure 11 doit être effectuée à une distance D au plus égale à 5 centimètres de la traverse 10.

Par ailleurs, une distance minimum de 15 centimètres doit être respectée dans deux mesures de compacité 11.

En outre, il est préférable que chaque mesure 11 soit située à une distance au plus égale à 5 centimètres du rail 12.

En référence à la figure 2, chaque mesure 11 est effectuée du coté extérieur du rail 12 à proximité de l'intersection entre la traverse 10 et le rail 12. Par conséquent, selon ce mode de réalisation, quatre mesures de compacité sont réalisées à proximité d'une même traverse 10.

Ces quatre mesures sont moyennées de façon à déterminer la résistance de pointe moyenne $Qd_{moy}$ à proximité de la traverse considérée et ainsi à évaluer le tassement de cette traverse 10.

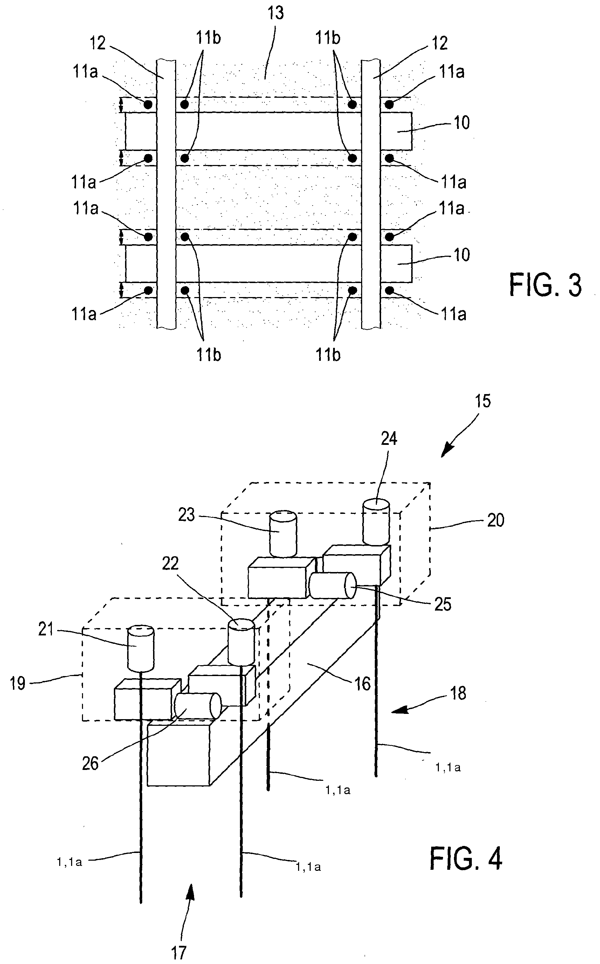

Selon une seconde variante du procédé de l'invention et en référence à la figure 3, huit mesures de la résistance de pointe du ballast sont prises à proximité d'une même traverse 10.

Quatre premières mesures référencées 11a sur la figure 3 correspondant à quatre points d'impact 11a du pénétromètre 1 sont effectuées comme pour la première variante du coté extérieur du rail 12 à proximité de l'intersection entre la traverse 10 et le rail 12. Quatre secondes mesures référencées 11b sont effectuées du coté intérieur au rail 12 à proximité de l'intersection entre la traverse 10 et le rail 12. Ces huit mesures 11a,11b, respectent également une distance maximum de 5 centimètres avec la traverse 10.

Elles sont par ailleurs espacées d'au moins 15 centimètres les unes des autres et situées à une distance d de 5 centimètres maximum du rail 12.

Selon l'invention, au moins deux mesures de la résistance de pointe $Qd$ doivent être effectuées à proximité d'une même traverse 10. Dans le cas où deux mesures de résistance de pointe $Qd$ sont prises, elles doivent l'être de part et d'autre de la traverse pour pouvoir estimer le plus précisément possible l'état de compacité du ballast sous cette traverse.

Par ailleurs, quel que soit le nombre mesures de résistance de pointe, ces mesures peuvent être prises soit simultanément, soit successivement.

Ainsi, selon l'invention, plusieurs mesures de la résistance de pointe $Qd$ du ballast à proximité d'une traverse 10 permettent de définir le degré de compacité du ballast 13 sous les traverses 10. La localisation de ces mesures telle que précédemment définie est importante car elles doivent donner une estimation de l'état mécanique du ballast 13 sous chaque traverse 10.

Selon l'invention, ces mesures de résistance peuvent être effectuées par un dispositif illustré sur la figure 4 et pouvant être monté sur tout appareil de voie.

En référence à cette figure, le dispositif 15 comporte un bâti 16 de part et d'autre duquel s'étendent verticalement quatre pénétromètres 1a de type statique. La largeur du bâti 16 ainsi que le positionnement relatif des pénétromètres 1 sont ajustés de façon que lorsque le bâti 16 est disposé en regard vertical d'une traverse non visible sur cette figure, les points d'impact des pénétromètres 1a respectent les conditions précédemment définies concernant notamment la distance entre chaque point d'impact des pénétromètres 1a et la traverse considérée.

Le dispositif représenté sur la figure 4 comportant quatre pénétromètres 1a, leur disposition relative sera celle permettant de réaliser quatre mesures 11 telles que définies dans la première variante représentée sur la figure 2.

Chaque couple 17,18 de pénétromètre 1 destinés à effectuer des mesures de compacité au niveau d'un même rail, sont surmontés d'un bloc d'enfoncement 19,20. Chaque bloc d'enfoncement 19,20 comporte deux vérins hydrauliques, pneumatiques d'enfoncement 21,22 ;23,24 destinés à appliquer l'énergie d'enfoncement sur chaque pénétromètre 1a.

Par ailleurs, chaque bloc d'enfoncement 19,20 comporte un vérin hydraulique de positionnement latéral 25,26 qui permettent de positionner latéralement le pénétromètre 1a auquel ils sont reliés. L'actionnement de chaque vérin de positionnement latéral 25, 26 est effectué au moyen d'une encoche non visible réalisée sur la face latérale du bloc d'enfoncement considéré 19,20. Ainsi, la distance entre les points d'impact des pénétromètres 1a et la traverse considérée est contrôlée.

Le procédé de l'invention consiste ainsi notamment à effectuer au moins deux mesures de la compacité du ballast à proximité d'une traverse au moyen de pénétromètres mesurant la résistance de pointe.

Une fois que ces mesures de résistance de pointe $Qd$ sont effectuées, un calculateur détermine la moyenne de ces mesures pour déterminer la résistance de pointe moyenne $Qd_{moy}$ sous une traverse, ce qui définit l'état du ballast sous la traverse considérée.

La méthode de prédiction du tassement d'une couche de ballast de voie ferrée de l'invention comporte une seconde étape consistant à déterminer l'évolution dans le temps de l'état du ballast sous chaque traverse.

A cet effet, la Demanderesse a adapté la loi de relaxation de la densité connue pour s'appliquer pour des billes de verre mono-disperses, à la compacité du ballast selon le nombre de passages d'essieux sur les traverses étudiées.

La loi de relaxation modifiée prend alors la forme suivante :

$$\tau_N = \tau_\infty \left( 1 - \frac{1}{1 + B \ln\left(1 + \dfrac{N}{No}\right)} \right)$$

où $\tau_N$ traduit l'évolution du tassement de la couche de ballast exprimé en millimètre en fonction de $N$ qui est le nombre de passage d'essieux de véhicule ferroviaire sur la traverse considérée,

où $No$ répond à la formule suivante :

$$No = a \times \ln\left( \frac{Qd_{0\,moy}}{p} \right) + b$$

où $a$ est compris entre 46 et 57, et $b$ est compris entre 40 et 50,
où $Qd_{0\,moy}$ est la résistance de pointe moyenne pour une traverse déterminée telle que précédemment décrit, et prise à l'issue d'opérations de maintenance ou lors de l'installation de la voie ferrée, et où $p$ est la pression sous la traverse considérée, exprimé en Mégapascal,

où $\tau_\infty$ répond à la formule suivante :

$$\tau_\infty = Ho\left( c \times \Gamma + e \right)$$

où $c$ est compris entre 21 et 32 et $e$ est compris entre 0,0040 et 0,006,
où $Ho$ est l'épaisseur de la couche de ballast considérée exprimée en millimètres, et
où $\Gamma$ est l'intensité réduite des vibrations auquel est soumis le ballast à proximité de la traverse considérée et répond à la formule suivante :

$$\Gamma = \frac{Aw^2}{\dfrac{pd^2}{m} + g}$$

où $A$ est l'amplitude de l'enfoncement vertical élastique de la voie, calculée à partir d'un modèle mécanique ou mesurée, exprimée en mètre,
où $w^2 = 2\pi f$, f étant la fréquence de passage des bogies en Hertz,
où $p$ est l'effort appliqué par la traverse considérée sur le ballast, exprimée en pascals,
où $d$ est le diamètre moyen d'un grain du ballast exprimé en mètre, et

où *m* est la masse d'un grain du ballast exprimée en kilogramme, et

où *B* répond à la formule suivante :

$$B = h \times \Gamma^i$$

où *h* est compris entre 0,0003 et 0,0014, et *i* est compris entre - 0,3 et - 1,15, et où $\Gamma$ est l'intensité réduite des vibrations auquel est soumis le ballast à proximité de la traverse considérée.

Par conséquent, l'évolution du tassement de la couche de ballast d'une voie ferrée peut être appréhendée de façon à pouvoir intervenir sur la voie lorsque le tassement du ballast, telle que déterminé par la loi de tassement précitée de l'invention, devient trop excessif.

## Revendications

1. Procédé d'évaluation de la compacité d'une couche de ballast de voie ferrée à proximité d'une traverse, **caractérisé en ce qu'**il comporte au moins une étape de prise d'au moins deux mesures (11,11a,11b) de la résistance de pointe (*Qd*) du ballast (13) à proximité d'une même traverse (10), et une étape de calcul de la valeur moyenne (*Qd_{moy}*) de ces mesures (11,11a,11b) de résistance de pointe (*Qd*), **en ce que** chaque mesure de la résistance de pointe (*Qd*) est réalisée au moyen d'un pénétromètre (1,1a) qui est enfoncé dans le sol (5) par des opérations d'enfoncement successives au cours de chacune desquelles est obtenue une valeur de résistance de pointe instantanée (*Qd_i*) du ballast correspondant à chaque enfoncement, et **en ce que** l'on détermine ladite résistance de pointe (*Qd*) en moyennant toutes les valeurs de résistance de pointe instantanées (*Qd_i*) obtenues précédemment.

2. Procédé selon la revendication 1, **caractérisé en ce que** chaque mesure (11,11a,11b) de résistance de pointe (*Qd*) est prise à une distance maximum de cinq centimètres de la traverse (10) et de part et d'autre de la dite traverse (10).

3. Procédé selon l'une quelconque des revendications 1 et 2, **caractérisé en ce que** les mesures (11,11a,11b) de résistance de pointe (*Qd*) sont espacées d'au moins quinze centimètres les unes des autres.

4. Procédé selon l'une quelconque des revendications 1 à 3, **caractérisé en ce que** chaque mesure (11,11a,11b) de résistance de pointe (*Qd*) est prise à une distance maximum de cinq centimètres du rail (12).

5. Procédé selon l'une quelconque des revendications 1 à 4, **caractérisé en ce qu'**il comporte au moins une étape de prise de quatre mesures (11) de la résistance de pointe (*Qd*) du ballast (13) à proximité d'une même traverse (10), chaque mesure (11) étant effectuée du coté extérieur du rail (12), de part et d'autre de la traverse (10) et à proximité de l'intersection entre la traverse (10) et le rail (12).

6. Procédé selon l'une quelconque des revendications 1 à 4, **caractérisé en ce qu'**il comporte au moins une étape de prise de huit mesures (11a,11b) de la résistance de pointe (*Qd*) du ballast (13) à proximité d'une même traverse (10), quatre première mesures (11a) étant effectuées de part et d'autre de la traverse (10) du coté extérieur du rail (12) à proximité de l'intersection entre la traverse (10) et le rail (12), et quatre secondes mesures (11b) étant effectuées du coté intérieur au rail (12) à proximité de l'intersection entre la traverse (10) et le rail (12).

7. Procédé selon l'une quelconque des revendications précédentes, **caractérisé en ce que** chaque mesure (11,11a,11b) de la résistance de pointe (*Qd*) du ballast (13) à proximité d'une traverse (10) est réalisée au moyen d'un pénétromètre statique (1a) pour lequel l'enfoncement du pénétromètre (1a) est réalisé au moyen d'un vérin d'enfoncement vertical (21,22,23,24), et **en ce qu'**un calculateur détermine la résistance de pointe instantanée (*Qd_i*) par la formule suivante :

$$Qd_i = \frac{F}{A}$$

où *Qd_i* est la résistance de pointe instantanée par enfoncement du pénétromètre,
*A* est la section de la pointe exprimée en mètres carrés, et
F est la force de poussée exercée sur le pénétromètre exprimée en newton,
et **en ce que** le calculateur moyenne toutes les valeurs de résistance de pointe instantanées (*Qd_i*) obtenues pour déterminer la résistance de pointe (*Qd*).

8. Procédé selon l'une quelconque des revendications 1 à 6, **caractérisé en ce que** chaque mesure (11,11a,11b) de la résistance de pointe (*Qd*) du ballast (13) à proximité d'une traverse (10) est réalisée au moyen d'un pénétromètre dynamique (1) comportant une tête de battage (2) sur laquelle est appliquée au moins une chute de masse qui entraîne l'enfoncement dans le ballast (13) d'une pointe conique (2) située à l'extrémité opposée de la tête de battage (1) et reliée à cette dernière par une tige (3), et **en ce qu'**un calculateur (8) détermine la résistance de pointe instantanée du ballast (13) par la formule suivante :

$$Qd_i = \frac{E}{A \times e}$$

où $Qd_i$ est la résistance de pointe instantanée pour un battage

$E$ est l'énergie reçue sur la tête de battage exprimée en joules,

$A$ est la section de la pointe exprimée en mètres carrés,

$e$ est la profondeur d'enfoncement de la pointe conique exprimée en mètres,

et **en ce que** le calculateur moyenne toutes les valeurs de résistance de pointe instantanées ($Qd_i$) obtenues pour déterminer la résistance de pointe ($Qd$).

9. Méthode de prédiction du tassement d'une couche de ballast de voie ferrée, **caractérisée en ce qu'**elle comporte au moins une étape d'évaluation de la compacité d'une couche de ballast (13) de voie ferrée par la détermination de la valeur moyenne de la résistance de pointe ($Qd_{0\ moy}$) du ballast (13) à proximité d'une traverse (10) selon le procédé de l'une quelconque des revendications 1 à 7, et une étape de prédiction du tassement de la dite couche de ballast (13) par application de la formule suivante :

$$\tau_N = \tau_\infty \left( 1 - \frac{1}{1 + B \ln\left(1 + \frac{N}{No}\right)} \right)$$

où $\tau_N$ traduit l'évolution du tassement de la couche de ballast en fonction de $N$ qui est le nombre de passage d'essieux sur la traverse considérée,

où $No$ répond à la formule suivante :

$$No = a \times \ln\left(\frac{Qd_{0\ moy}}{p}\right) + b$$

où $a$ est compris entre 46 et 57, et $b$ est compris entre 40 et 50,

où $Qd_{0\ moy}$ est la résistance de pointe déterminée à l'étape d'évaluation de la compacité de la couche de ballast, et prise à l'issue d'opérations de maintenance ou lors de l'installation de la voie ferrée, et

où $p$ est la pression sous la traverse considérée exprimée en mégapascal

où $\tau_\infty$ répond à la formule suivante :

$$\tau_\infty = Ho\left(c \times \Gamma + e\right)$$

où $c$ est compris entre 21 et 32, et $e$ est compris entre 0,0040 et 0,006,

où $Ho$ est l'épaisseur de la couche de ballast considérée exprimée en millimètres, et

où $\Gamma$ est l'intensité réduite des vibrations auquel est soumis le ballast à proximité de la traverse considérée et répond à la formule suivante :

$$\Gamma = \frac{Aw^2}{\frac{pd^2}{m} + g}$$

où $A$ est l'amplitude de l'enfoncement vertical élastique de la voie, calculée à partir d'un modèle mécanique ou mesurée, exprimée en Mètres,

où $w^2 = 2\pi f$, f étant la fréquence de passage des bogies en Hertz

où $p$ est l'effort appliqué par la traverse considérée sur le ballast exprimée en Pascal,

où $d$ est le diamètre moyen d'un grain du ballast exprimée en Mètre, et

où $m$ est la masse d'un grain du ballast exprimée en Kilogramme, et

où $B$ répond à la formule suivante :

$$B = h \times \Gamma^i$$

où $h$ est compris entre 0,0003 et 0,0014, et $i$ est compris entre - 0,3 et - 1,15,

où $\Gamma$ est l'intensité réduite des vibrations auquel est soumis le ballast à proximité de la traverse considérée.

10. Dispositif pour mettre en oeuvre le procédé d'évaluation de la compacité d'une couche de ballast de voie ferrée à proximité d'une traverse selon l'une quelconque des revendications 1 à 7, **caractérisé en ce qu'**il comporte au moins deux pénétromètres statiques (1a) aptes à être enfoncés dans le ballast (13) à proximité d'une même traverse, l'enfoncement de chaque pénétromètre (1a) étant réalisé au moyen d'un vérin d'enfoncement vertical (21,22,23,24), et **en ce qu'**il comporte un calculateur déterminant la résistance de pointe instantanée (Q$_{di}$) par la formule suivante :

$$Qd_i = \frac{F}{A}$$

où $Qd_i$ est la résistance de pointe instantanée par enfoncement d'un pénétromètre,
$A$ est la section de la pointe en mètres carrés, et F est la force de poussée exercée sur le pénétromètre exprimée en Newton, **en ce que** le calculateur moyenne toutes les valeurs de résistance de pointe instantanées ($Qd_i$) obtenues pour chaque pénétromètre pour déterminer la résistance de pointe (Qd) de chaque pénétromètre, et **en ce que** le calculateur moyenne toutes ces valeurs de résistance de pointe ($Qd$) obtenues pour déterminer la résistance de pointe moyenne ($Qd_{moy}$) du ballast sous une traverse.

11. Dispositif selon la revendication 10, **caractérisé en ce qu'**il comporte un bâti (16) destiné à être positionné en regard verticalement et à distance de la voie ferrée, et comprenant au moins quatre pénétromètres (1a) fixés sur le dit bâti (16) et dont les positions relatives permettent de réaliser quatre mesures (11,11a,11b) de la résistance de pointe ($Qd$) du ballast (13) à proximité d'une même traverse (10).

12. Dispositif selon l'une quelconque des revendications 10 et 11, **caractérisé en ce que** chaque vérin d'enfoncement vertical (21,22,23,24) est apte à s'enfoncer dans le ballast à une vitesse d'enfoncement inférieure à 3 centimètres par seconde.

13. Dispositif selon l'une quelconque des revendications 10 à 12, **caractérisé en ce qu'**au moins deux pénétromètres (1a) destinés à mesurer la résistance de pointe sur le même côté d'une traverse (10) sont agencés avec un vérin de positionnement latéral (25,26) qui permet de contrôler la distance entre les points d'impact desdits pénétromètres (1a) et le traverse (10) considérée.

14. Dispositif pour mettre en oeuvre le procédé d'évaluation de la compacité d'une couche de ballast de voie ferrée à proximité d'une traverse selon l'une quelconque des revendications 1 ou 8, **caractérisé en ce qu'**il comporte au moins un pénétromètre dynamique (1) apte à être enfoncé dans le ballast (13) en au moins deux points à proximité d'une même traverse, **en ce qu'**il comporte un calculateur déterminant la résistance de pointe instantanée (Q$_{di}$) par la formule suivante :

$$Qd_i = \frac{E}{A \times e}$$

où $Qd_i$ est la résistance de pointe instantanée pour un battage
$E$ est l'énergie reçue sur la tête de battage exprimée en joules,
$A$ est la section de la pointe exprimée en mètres carrés, et $e$ est la profondeur d'enfoncement de la pointe conique dans le sol (5) exprimée en mètres, **en ce que** le calculateur moyenne toutes les valeurs de résistance de pointe instantanées ($Qd_i$) obtenues pour chaque point d'enfoncement pour déterminer la résistance de pointe (Qd) à chaque point d'enfoncement, et **en ce que** le calculateur moyenne toutes ces valeurs de résistance de pointe ($Qd$) obtenues pour déterminer la résistance de pointe moyenne ($Qd_{moy}$) du ballast sous une traverse.

**Patentansprüche**

1. Verfahren zur Beurteilung der Kompaktheit einer Schotterschicht einer Eisenbahnstrecke in der Nähe einer Schwelle,
**dadurch gekennzeichnet, dass** dieses mindestens einen Schritt der Abnahme von mindestens zwei Messungen (11, 11a, 11b) des Sollpunktwiderstands (Qd) des Schotters (13) in der Nähe derselben Schwelle (10) und einen Schritt der Berechnung des Mittelwerts (Qd$_{moy}$) dieser Messungen (11, 11a, 11b) des Sollpunktwiderstands (Qd) umfasst, dadurch, dass jede Messung des Sollpunktwiderstands (Qd) mit einem Penetrometer (1, 1a) durchgeführt wird, das in den Boden (5) durch aufeinanderfolgende Eindrückvorgänge eingedrückt wird, während jedes von welchen ein Wert des momentanen Sollpunktwiderstands (Qd$_i$) des Schotters, der jedem Eindrücken entspricht, erhalten wird, und dadurch, dass der Sollpunktwiderstand (Qd) durch Mittelung aller zuvor erhaltenen Werte des momentanen Sollpunktwiderstands (Qd$_i$) bestimmt wird.

2. Verfahren nach Anspruch 1,
**dadurch gekennzeichnet, dass** jede Messung (11, 11a, 11b) des Sollpunktwiderstands (Qd) in einer maximalen Distanz von fünf Zentimetern von der Schwelle (10) auf beiden Seiten der Schwelle (10) abgenommen wird.

3. Verfahren nach einem der Ansprüche 1 und 2,
**dadurch gekennzeichnet, dass** die Messungen (11, 11a, 11b) des Sollpunktwiderstands (Qd) mindestens fünfzehn Zentimeter voneinander beabstandet werden.

4. Verfahren nach einem der Ansprüche 1 bis 3, **dadurch gekennzeichnet, dass** jede Messung (11, 11a, 11b) des Sollpunktwiderstands (Qd) in einer maximalen Distanz von fünf Zentimetern von der Schiene (12) abgenommen wird.

5. Verfahren nach einem der Ansprüche 1 bis 4, **dadurch gekennzeichnet, dass** dieses mindestens einen Schritt der Abnahme von vier Messungen (11) des Sollpunktwiderstands (Qd) des Schotters (13) in der Nähe derselben Schwelle (10) umfasst, wobei jede Messung (11) an der Außenseite der Schiene (12) auf beiden Seiten der Schwelle (10) und in der Nähe des Schnittpunkts zwischen der Schwelle (10) und der Schiene (12) durchgeführt wird.

6. Verfahren nach einem der Ansprüche 1 bis 4, **dadurch gekennzeichnet, dass** dieses mindestens einen Schritt der Abnahme von acht Messungen (11a, 11b) des Sollpunktwiderstands (Qd) des Schotters (13) in der Nähe derselben Schwelle (10) umfasst, wobei vier erste Messungen (11a) auf beiden Seiten der Schwelle (10) an der Außenseite der Schiene (12) in der Nähe des Schnittpunkts zwischen der Schwelle (10) und der Schiene (12) durchgeführt werden, und vier zweite Messungen (11b) an der Innenseite der Schiene (12) in der Nähe des Schnittpunkts zwischen der Schwelle (10) und der Schiene (12) durchgeführt werden.

7. Verfahren nach einem der vorhergehenden Ansprüche,
**dadurch gekennzeichnet, dass** jede Messung (11, 11a, 11b) des Sollpunktwiderstands (Qd) des Schotters (13) in der Nähe einer Schwelle (10) mit einem statischen Penetrometer (1a) durchgeführt wird, für welches das Eindrücken des Penetrometers (1a) mit einem vertikalen Eindrückzylinder (21, 22, 23, 24) durchgeführt wird, und dadurch, dass ein Rechner den momentanen Sollpunktwiderstand ($Qd_i$) durch die folgende Formel bestimmt:

$$QQ_Q = \frac{Q}{Q}$$

wobei $Qd_i$ der momentane Sollpunktwiderstand durch Eindrücken des Penetrometers ist,
A der Querschnitt des Punkts ist, ausgedrückt in Quadratmeter, und
F die Druckkraft ist, die auf das Penetrometer ausgeübt wird, ausgedrückt in Newton,
und dadurch, dass der Rechner alle Werte des momentanen Sollpunktwiderstands ($Qd_i$) mittelt, die erhalten werden, um den Sollpunktwiderstand (Qd) zu bestimmen.

8. Verfahren nach einem der Ansprüche 1 bis 6, **dadurch gekennzeichnet, dass** jede Messung (11, 11a, 11b) des Sollpunktwiderstands (Qd) des Schotters (13) in der Nähe einer Schwelle (10) mit einem dynamischen Penetrometer (1) durchgeführt wird, das einen Rammkopf (2) umfasst, auf dem mindestens ein Fallgewicht angebracht ist, welches das Eindrücken, in den Schotter (13), einer konischen Spitze (2) bewirkt, die am gegenüberliegenden Ende des Rammkopfs (1) angeordnet ist und mit diesem Letzteren durch einen Schaft (3) verbunden ist, und dadurch, dass ein Rechner (8) den momentanen Sollpunktwiderstand des Schotters (13) durch die folgende Formel bestimmt:

$$QQ_Q = \frac{Q}{QQQ}$$

wobei $Qd_i$ der momentane Sollpunktwiderstand für ein Rammen ist,
E die Energie ist, die am Rammkopf empfangen wird, ausgedrückt in Joule,
A der Querschnitt des Punkts ist, ausgedrückt in Quadratmeter, und
e die Eindrücktiefe der konischen Spitze ist, ausgedrückt in Meter,
und dadurch, dass der Rechner alle Werte des momentanen Sollpunktwiderstands ($Qd_i$) mittelt, die erhalten werden, um den Sollpunktwiderstand (Qd) zu bestimmen.

9. Verfahren zur Vorhersage der Verdichtung einer Schotterschicht einer Eisenbahnstrecke,
**dadurch gekennzeichnet, dass** dieses mindestens einen Schritt der Beurteilung der Kompaktheit einer Schotterschicht (13) der Eisenbahnstrecke durch die Bestimmung des Mittelwerts des Sollpunktwiderstands ($Qd_{0moy}$) des Schotters (13) in der Nähe einer Schwelle (10) gemäß dem Verfahren nach einem der Ansprüche 1 bis 7 umfasst, und einen Schritt der Vorhersage der Verdichtung der Schotterschicht (13) durch Anwenden der folgenden Formel:

$$\tau_N = \tau_\infty \left( 1 - \frac{1}{1 + B \ln\left(1 + \frac{N}{No}\right)} \right)$$

wobei $\tau_N$ die Entwicklung der Verdichtung der Schotterschicht als Funktion von N darstellt, das die Anzahl von Achsdurchläufen auf der betreffenden Schwelle ist,
wobei No die folgende Formel aufweist:

$$No = a \times \ln\left(\frac{Qd_{0\,moy}}{p}\right) + b$$

wobei a zwischen 46 und 57 beträgt, und b zwischen 40 und 50 beträgt,
wobei $Qd_{0moy}$ d der Sollpunktwiderstand ist, der im Schritt der Beurteilung der Kompaktheit der Schotterschicht bestimmt wird, und nach Wartungsvorgängen oder während der Installation der Eisenbahnstrecke abgenommen wird, und wobei p der Druck unter der betreffenden Schwelle ist, ausgedrückt in Megapascal, wobei $\tau_\infty$ die folgende Formel aufweist:

$$\tau_\infty = \text{Ho}(\text{cx}\Gamma + \text{e})$$

wobei c zwischen 21 und 32 beträgt, und e zwischen 0,0040 und 0,006 beträgt,
wobei Ho die Dicke der betreffenden Schotterschicht ist, ausgedrückt in Millimeter, und wobei $\Gamma$ die reduzierte Intensität von Vibrationen ist, denen der Schotter in der Nähe der betreffenden Schwelle ausgesetzt ist, und die folgende Formel aufweist:

$$\Gamma = \frac{Aw^2}{\dfrac{pd^2}{m} + g}$$

wobei A die Amplitude des elastischen vertikalen Eindrückens der Strecke ist, berechnet aus einem mechanischen Modell oder gemessen, ausgedrückt in Meter,
wobei $w^2 = 2\pi f$, wobei f die Drehdurchlauffrequenz in Hertz ist,
wobei p die Kraft ist, die von der betreffenden Schwelle auf den Schotter ausgeübt wird, ausgedrückt in Pascal,
wobei d der mittlere Durchmesser eines Schotterkorns ist, ausgedrückt in Meter, und wobei m die Masse eines Schotterkorns ist, ausgedrückt in Kilogramm, und wobei B die folgende Formel aufweist:

$$\text{B} = \text{hx}\Gamma^{\text{i}}$$

wobei h zwischen 0,0003 und 0,0014 beträgt, und i zwischen -0,3 und -1,15 beträgt,
wobei $\Gamma$ die reduzierte Intensität von Vibrationen ist, denen der Schotter in der Nähe der betreffenden Schwelle ausgesetzt ist.

10. Vorrichtung zur Durchführung des Verfahrens zur Beurteilung der Kompaktheit einer Schotterschicht einer Eisenbahnstrecke in der Nähe einer Schwelle nach einem der Ansprüche 1 bis 7,
**dadurch gekennzeichnet, dass** diese mindestens zwei statische Penetrometer (1a) umfasst, die geeignet sind, in den Schotter (13) in der Nähe derselben Schwelle eingedrückt zu werden, wobei das Eindrücken jedes Penetrometers (1a) mit einem vertikalen Eindrückzylinder (21, 22, 23, 24) durchgeführt wird, und dadurch, dass diese einen Rechner umfasst, der den momentanen Sollpunktwiderstand ($Qd_i$) durch die folgende Formel bestimmt:

$$QQ_Q = \frac{Q}{Q}$$

wobei $Qd_i$ der momentane Sollpunktwiderstand durch Eindrücken eines Penetrometers ist,
A der Querschnitt des Punkts in Quadratmeter ist, und
F die Druckkraft ist, die auf das Penetrometer ausgeübt wird, ausgedrückt in Newton,
und dadurch, dass der Rechner alle Werte des momentanen Sollpunktwiderstands ($Qd_i$) mittelt, die für jedes Penetrometer erhalten werden, um den Sollpunktwiderstand ($Qd$) jedes Penetrometers zu bestimmen, und dadurch, dass der Rechner all diese Werte des Sollpunktwiderstands ($Qd$) mittelt, die erhalten werden, um den mittleren Sollpunktwiderstand ($Qd_{moy}$) des Schotters unter einer Schwelle zu bestimmen.

11. Vorrichtung nach Anspruch 10,
**dadurch gekennzeichnet, dass** diese ein Gestell (16) umfasst, das dazu bestimmt ist, vertikal gegenüber und in einer Distanz von der Eisenbahnstrecke positioniert zu werden, und mindestens vier Penetrometer (1a) umfasst, die an dem Gestell (16) befestigt sind, und deren relative Positionen gestatten, vier Messungen (11, 11a, 11b) des Sollpunktwiderstands (Qd) des Schotters (13) in der Nähe derselben Schwelle (10) durchzuführen.

12. Vorrichtung nach einem der Ansprüche 10 und 11,
**dadurch gekennzeichnet, dass** jeder Eindrückzylinder (21, 22, 23, 24) geeignet ist, in den Schotter mit einer kleineren Eindrückgeschwindigkeit als 3 Zentimeter pro Sekunde eingedrückt zu werden.

13. Vorrichtung nach einem der Ansprüche 10 bis 12,
**dadurch gekennzeichnet, dass** mindestens zwei Penetrometer (1a), die dazu bestimmt sind, den Sollpunktwiderstand auf derselben Seite einer Schwelle (10) zu messen, mit einem lateralen Positionierungszylinder (25, 26) versehen sind, der es gestattet, die Distanz zwischen den Auftreffpunkten der Penetrometer (1a) und der betreffenden Schwelle (10) zu steuern.

14. Vorrichtung zur Durchführung des Verfahrens zur Beurteilung der Kompaktheit einer Schotterschicht einer Eisenbahnstrecke in der Nähe einer Schwelle nach einem der Ansprüche 1 oder 8, **dadurch gekennzeichnet, dass** diese mindestens ein dynamisches Penetrometer (1) umfasst, das geeignet ist, in den Schotter (13) an mindestens zwei Punkten in der Nähe derselben Schwelle eingedrückt zu werden, dadurch, dass diese einen Rechner umfasst, der den momentanen Sollpunktwiderstand (Qd_i) durch die folgende Formel bestimmt:

$$QQ_Q = \frac{Q}{QQQ}$$

wobei $Qd_i$ der momentane Sollpunktwiderstand für ein Rammen ist,
E die Energie ist, die am Rammkopf empfangen wird, ausgedrückt in Joule,
A der Querschnitt des Punkts ist, ausgedrückt in Quadratmeter, und
e die Eindrücktiefe der konischen Spitze in den Boden (5) ist, ausgedrückt in Meter,
dadurch, dass der Rechner alle Werte des momentanen Sollpunktwiderstands (Qd_i) mittelt, die für jeden Eindrückpunkt erhalten werden, um den Sollpunktwiderstand (Qd) an jedem Eindrückpunkt zu bestimmen, und dadurch, dass der Rechner all diese Werte des Sollpunktwiderstands (Qd) mittelt, die erhalten werden, um den mittleren Sollpunktwiderstand (Qd_moy) des Schotters unter einer Schwelle zu bestimmen.

**Claims**

1. Method for evaluating the compactness of a layer of railway line ballast in the vicinity of a sleeper, **characterized in that** it comprises at least a step of taking at least two measurements (11, 11a, 11b) of the tip resistance (*Qd*) of the ballast (13) in the vicinity of the one same sleeper (10), and a step of calculating the mean value (*Qd_moy*) of these tip resistance (*Qd*) measurements (11, 11a, 11b), **in that** each tip resistance (*Qd*) measurement is performed using a penetrometer (1, 1a) which is driven into the ground (5) by successive driving operations during each of which a value is obtained for the instantaneous tip resistance (*Qd_i*) of the ballast corresponding to each driving operation, and **in that** the said tip resistance (*Qd*) is determined by averaging all the instantaneous tip resistance (*Qd_i*) values obtained previously.

2. Method according to Claim 1, **characterized in that** each tip resistance (*Qd*) measurement (11, 11a, 11b) is taken at a maximum distance of five centimetres away from the sleeper (10) and on either side of the said sleeper (10).

3. Method according to either one of Claims 1 and 2, **characterized in that** the tip resistance (*Qd*) measurements (11, 11a, 11b) are spaced at least fifteen centimetres apart.

4. Method according to any one of Claims 1 to 3, **characterized in that** each tip resistance (*Qd*) measurement (11, 11a, 11b) is taken at a maximum distance of five centimetres from the rail (12).

5. Method according to any one of Claims 1 to 4, **characterized in that** it comprises at least a step of taking four measurements (11) of the tip resistance (*Qd*) of the ballast (13) in the vicinity of the one same sleeper (10), each measurement (11) being taken on the outside of the rail (12), on either side of the sleeper (10) and in the vicinity of the intersection between the sleeper (10) and the rail (12).

6. Method according to any one of Claims 1 to 4, **characterized in that** it comprises at least a step of taking eight measurements (11a, 11b) of the tip resistance (*Qd*) of the ballast (13) in the vicinity of the one same sleeper (10), four first measurements (11a) being taken on either side of the sleeper (10) on the outside of the rail (12) in the vicinity of the intersection between the sleeper (10) and the rail (12), and four second measurements (11b) being taken on the inside of the rail (12) in the vicinity of the intersection between the sleeper (10) and the rail (12).

7. Method according to any one of the preceding claims, **characterized in that** each measurement (11, 11a, 11b) of the tip resistance (*Qd*) of the ballast (13) in the vicinity of a sleeper (10) is taken by means of a static penetrometer (1a), for which the penetrometer (1a) is driven by means of a vertical driving ram (21, 22, 23, 24) and **in that** a computer determines the instantaneous tip resistance (*Qd_i*) using the following formula:

$$Qd_i = \frac{F}{A}$$

where $Qd_i$ is the instantaneous tip resistance per driving operation of the penetrometer,
A is the cross section of the tip expressed in square metres, and
F is the thrust force applied to the penetrometer, expressed in newtons,
and **in that** the computer averages all the instantaneous tip resistance (*Qd_i*) values obtained

in order to determine the tip resistance ($Qd$).

8. Method according to any one of Claims 1 to 6, **characterized in that** each measurement (11, 11a, 11b) of the tip resistance ($Qd$) of the ballast (13) in the vicinity of a sleeper (10) is taken using a dynamic penetrometer (1) comprising a striking head (2) to which there is applied at least one drop of a hammer, which drives into the ballast (13) a cone tip (2) situated at the opposite end of the striking head (1) and connected thereto by a rod (3), and **in that** a computer (8) determines the instantaneous tip resistance of the ballast (13) using the following formula:

$$Qd_i = \frac{E}{A \times e}$$

where $Qd_i$ is the instantaneous tip resistance for one strike
$E$ is the energy received on the striking head, expressed in joules,
$A$ is the cross section of the tip expressed in square metres,
$e$ is the depth to which the cone tip is driven, expressed in metres,
and **in that** the computer averages all the instantaneous tip resistance ($Qd_i$) values obtained in order to determine the tip resistance ($Qd$).

9. Method for predicting the compaction of a layer of railway line ballast, **characterized in that** it comprises at least a step of evaluating the compactness of a layer of railway line ballast (13) by determining the mean value of the tip resistance ($Qd_{0moy}$) of the ballast (13) in the vicinity of a sleeper (10) using the method according to any one of Claims 1 to 7, and a step of predicting the compaction of the said layer of ballast (13) by applying the following formula:

$$\tau_N = \tau_\infty \left( 1 - \frac{1}{1 + B \ln\left(1 + \dfrac{N}{No}\right)} \right)$$

where $\tau_N$ indicates the change in compaction of the layer of ballast as a function of $N$ which is the number of passes of axles over the sleeper concerned,
where $No$ satisfies the following formula:

$$No = a \times \ln\left(\frac{Qd_{0moy}}{p}\right) + b$$

where a is comprised between 46 and 57 and b is comprised between 40 and 50,
where $Qd_{0moy}$ is the tip resistance determined in the step of evaluating the compactness of the layer of ballast and taken at the end of maintenance operations or during the laying of the railway track, and
where $p$ is the pressure underneath the sleeper concerned, expressed in megapascals
where $\tau_\infty$ satisfies the following formula:

$$\tau_\infty = Ho\left(c \times \Gamma + e\right)$$

where c is comprised between 21 and 32, and e is comprised between 0.0040 and 0.006,
where $Ho$ is the thickness of the layer of ballast concerned, expressed in millimetres, and
where $\Gamma$ is the reduced intensity of the vibrations to which the ballast is subjected in the vicinity of the sleeper concerned and satisfies the following formula:

$$\Gamma = \frac{Aw^2}{\dfrac{pd^2}{m} + g}$$

where $A$ is the amplitude of the elastic vertical penetration of the track, calculated from a mechanical model or measured, and expressed in metres,
where $w^2 = 2\pi f$, f being the bogie passage frequency in hertz
where $p$ is the load applied by the sleeper concerned to the ballast, expressed in Pascals, where $d$ is the mean diameter of a grain of ballast expressed in metres, and
where $m$ is the mass of a grain of ballast, expressed in kilograms, and

where $B$ satisfies the following formula:

$$B = h \times \Gamma^i$$

where $h$ is comprised between 0.0003 and 0.0014, and $i$ is comprised between -0.3 and -1.15,
where $\Gamma$ is the reduced intensity of the vibrations to which the ballast is subjected in the vicinity of the sleeper concerned.

10. Device for implementing the method for evaluating the compactness of a layer of railway line ballast in the vicinity of a sleeper according to any one of Claims 1 to 7, **characterized in that** it comprises at least two static penetrometers (1a) able to be driven into the ballast (13) in the vicinity of the one same sleeper, each penetrometer (1a) being driven by means of a vertical driving ram (21, 22, 23, 24), and **in that** it comprises a computer determining the instantaneous tip resistance ($Q_{di}$) using the following formula:

$$Qd_i = \frac{F}{A}$$

where $Qd_i$ is the instantaneous tip resistance per driving operation of a penetrometer,
$A$ is the cross section of the tip expressed in square metres, and
F is the thrust force applied to the penetrometer, expressed in newtons,
and **in that** the computer averages all the instantaneous tip resistance values ($Qd_i$) obtained for each penetrometer in order to determine the tip resistance ($Qd$) of each penetrometer, and **in that** the computer averages all these tip resistance ($Qd$) values obtained in order to determine the mean tip resistance ($Qd_{moy}$) of the ballast under a sleeper.

11. Device according to Claim 10, **characterized in that** it comprises a framework (16) intended to be positioned vertically facing and some distance away from the railway line, and comprising at least four penetrometers (1a) fixed to the said framework (16) and the relative positions of which allow four measurements (11, 11a, 11b) of the tip resistance ($Qd$) of the ballast (13) to be taken in the vicinity of the one same sleeper (10).

12. Device according to either one of Claims 10 and 11, **characterized in that** each vertical driving ram (21, 22, 23, 24) is able to drive into the ballast at a penetration rate of less than 3 centimetres per second.

13. Device according to any one of Claims 10 to 12, **characterized in that** at least two penetrometers (1a) intended to measure the tip resistance on the one same side of a sleeper (10) are arranged with a lateral positioning ram (25, 26) that makes it possible to control the distance between the points of impact of the said penetrometers (1a) and the sleeper (10) concerned.

14. Device for implementing the method for evaluating the compactness of a layer of railway line ballast in the vicinity of a sleeper according to either one of Claims 1 and 8, **characterized in that** it comprises at least one dynamic penetrometer (1) able to be driven into the ballast (13) at at least two points in the vicinity of the one same sleeper, and **in that** it comprises a computer that determines the instantaneous tip resistance ($Q_{di}$) using the following formula:

$$Qd_i = \frac{E}{A \times e}$$

where $Qd_i$ is the instantaneous tip resistance for one strike
$E$ is the energy received on the striking head, expressed in joules,
$A$ is the cross section of the tip expressed in square metres, and
$e$ is the depth to which the cone tip into the ground (5) is driven, expressed in metres, and **in that** the computer averages all the instantaneous tip resistance ($Qd_i$) values obtained for each driving point in order to determine the tip resistance ($Qd$) at each driving point, and **in that** the computer averages all these tip resistance ($Qd$) values obtained in order to determine the mean tip resistance ($Qd_{moy}$) of the ballast under a sleeper.

FIG. 1

FIG. 2

FIG. 3

FIG. 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2299202
- FR 2145920
- FR 7602166
- FR 2817344